# EUROPÄISCHE PATENTSCHRIFT

(11) **EP 0 771 553 B1**
(45) Veröffentlichungstag und Bekanntmachung des Hinweises auf die Patenterteilung: **06.08.2003**
(21) Anmeldenummer: 96116809.3
(22) Anmeldetag: 18.10.1996
(51) Int. Cl.: A61F 9/00, A61B 17/32

(54) **Vorrichtung zur Hornhautchirurgie**
Device for corneal surgery
Dispositif de chirurgie cornéenne

(30) Priorität: 30.10.1995 DE 19540439
(43) Veröffentlichungstag der Anmeldung: 07.05.1997
(73) Patentinhaber: Schwind eye-tech-solutions GmbH & Co. KG, 63801 Kleinostheim (DE)
(72) Erfinder: Schwind, Herbert, 63801 Kleinostheim (DE)
(74) Vertreter: Hofstetter, Alfons J., Dr.rer.nat.

(56) Entgegenhaltungen:
- WO-A-93/09738
- DE-A- 3 433 581
- US-A- 4 674 503
- US-A- 4 903 695
- US-A- 5 133 726
- US-A- 5 312 394
- PALLIKARIS ET AL.: "Laser In Situ Keratomileusis", LASERS IN SURGERY AND MEDICINE, NEW YORK, US, -1990, Band 10, Nr. 5, Seiten 463 - 468

## Beschreibung

Die Erfindung betrifft eine Vorrichtung nach dem Oberbegriff des Patentanspruches 1.

Bei einer derartigen, aus der deutschen Patentschrift 34 33 581 (= US 4,662,370) bekannten Vorrichtung wird auf das zu behandelnde Auge ein mit Unterdruck fixierbarer Basisring aufgesetzt. Am Basisring ist ein Support über das Auge bewegbar geführt. In dem Support ist ein oszillierendes Messer enthalten, dessen oszillierende Schneidbewegung quer zur Vorschubrichtung des Supports erfolgt. Hierbei wird zur lamellierenden refraktiven Hornhautchirurgie der Krümmungsradius der Hornhaut eines Patientenauges in vorausbestimmter Form verkleinert oder vergrößert, indem eine Hornhautlamelle entlang einer Schnittlinie vollständig abgeschnitten und vom Auge abgenommen wird. Die verbleibende Hornhaut weist nach diesem Schnitt die gewünschte Krümmung für die refraktive Sehkorrektur auf. Die gewünschte Krümmung wird durch eine auf die Hornhautoberfläche aufgesetzte und entsprechend geformte Applanationsfläche bewirkt. Hierbei wird in Kauf genommen, daß zur Sehkorrektur ein relativ großflächiger Schnitt, dessen Schnittfläche endgültig verbleibt, an der Hornhaut durchgeführt wird.

Ferner ist es bekannt, nach dem vollständigen Abtrennen der Hornhautlamelle am freigelegten Gewebe der Hornhaut mit Hilfe von Laserstrahlen Korrekturen vorzunehmen und die abgetrennte Hornhautlamelle wieder auf das Auge aufzusetzen (US 4,840,175). Das genaue Wiederaufsetzen der entfernten Hornhautlamelle auf das behandelte Auge stellt hohe Anforderungen an das Geschick des Chirurgen. Außerdem ist es praktisch unmöglich, die entfernte Hornhautlamelle in exakt der gleichen Ausrichtung, die die Lamelle vor dem Entfernen auf dem Auge hatte, wieder auf die Hornhaut aufzusetzen.

Aus der WO 93/09738 A ist eine Vorrichtung zur automatischen Positionierung und Betätigung von chirurgischen Instrumenten für die refraktive keratische Chirurgie, insbesondere für die lamellare refraktive Chirurgie, bekannt. Dabei soll die beschriebene Vorrichtung die Durchführung chirurgischer Techniken mit extremer Sicherheit und hoher Reproduzierbarkeit gestatten. Die Vorrichtung umfasst dabei unter anderem eine waagerechte Führung mit einem darauf verschiebbar gelagerten Trageglied, einen ersten linearen Antrieb zur Verschiebung des Trageglieds sowie einen zweiten Antrieb, der durch eine Plattform getragen wird und gegenüber dieser senkrecht verstellbar ist. Eine flache, für die Berührung mit einem zu operierenden anatomischen Körperteil dimensionierte Platte ist mit dem zweiten Antrieb so verbunden, dass sie gegenüber der Plattform in senkrechter Richtung automatisch verstellbar ist.

Aufgabe der Erfindung ist es, eine Vorrichtung der eingangs genannten Art zu schaffen, mit welcher nach der Hornhautbehandlung an der Hornhautoberfläche die vor der Hornhautbehandlung vorhandenen Gegebenheiten mit einfachen Mitteln wieder herstellbar sind.

Diese Aufgabe wird erfindungsgemäß durch die kennzeichnenden Merkmale des Patentanspruches 1 gelöst.

Dadurch, daß während des Schneidvorgangs die Vorschubbewegung des Messers an einer bestimmten Stelle in der Vorrichtung angehalten wird, erreicht man, daß nicht die gesamte Hornhautlamelle vom Auge entfernt wird. Die abgetrennte Hornhautlamelle ist bei der Erfindung über ein vom Hornhautgewebe gebildetes Scharnier mit der am Auge verbliebenen Hornhaut verbunden. Die Hornhautlamelle kann von der Hornhautoberfläche weggeklappt werden, so daß das Stroma für beispielsweise eine Laserbehandlung freilegbar ist.

Beim Rückhub des Supports, welcher die Schneidvorrichtung trägt, bleibt der Basisring am Auge bevorzugt durch den Unterdruck fixiert. In bevorzugter Weise wird auch beim Rückhub des Supports und der Schneideinrichtung der Unterdruck in der Ringkammer im Bereich der Hornhaut aufrechterhalten, so daß eine einwandfreie Fixierung der abgetrennten Hornhautlamelle während des Rückhubes der Schneidvorrichtung, insbesondere der Schneidklinge, erzielt wird. Der Antrieb der oszillierenden Messerbewegung ist beim Rückhub abgeschaltet. Aus der U.S.-Patentschrift 5,133,726 ist eine Schneidvorrichtung bekannt, bei welcher der Antrieb des oszillierenden Messers und der Antrieb des Supports miteinander gekoppelt ist, so daß eine getrennte Steuerung der Antriebe nicht möglich ist. Dies würde sich vor allem nachteilig beim Rückhub der Schneideinrichtung, bei welcher gemäß der Erfindung die Messerbewegung abgeschaltet werden muß, auswirken. Sobald der Rückhub beendet ist, wird der Unterdruck abgeschaltet, so daß die Schneidvorrichtung vom Auge entfernt werden kann und eine nachfolgende Behandlung, insbesondere des Stromas, nach dem Wegklappen der Hornhautlamelle um die verbliebene Scharnierstelle durchgeführt werden kann.

In bevorzugter Weise ist daher die Mitte des Basisringes, an welchem das Messer mittels des verschiebbaren Supportes geführt ist, in den Strahlengang eines auf das Patientenauge richtbaren Laserstrahls bewegbar. Hierdurch läßt sich eine Kombination der Schneidvorrichtung mit einem Gerät zur Durchführung einer Laserablatio an der Augenhornhaut kombinieren derart, daß zunächst die Hornhautlamelle teilweise unter Belassung der Scharnierstelle vom Auge getrennt und zurückgeklappt wird. Nach Entfernen der Schneideinrichtung vom Auge wird der Laserstrahl auf das freigelegte Stroma der Augenhornhaut gerichtet. Es kann dann die gewünschte Refraktionskorrektur am Auge durchgeführt werden. Nach der Laserbehandlung wird die weggeklappte Hornhautlamelle wieder auf das behandelte Stroma zurückgeklappt, wobei die zurückgeklappte Lamelle aufgrund des Scharnieres wieder in die Position zurückkommt, welche sie vor der Operation eingenommen hat. Dies ist von Bedeutung, weil die Oberfläche der Augenhornhaut nicht exakt eine Kugelkalotte bildet. Für eine genaue Refraktionskorrektur ist es daher erforderlich, daß die abgetrennte Hornhautlamelle wieder mit der Positionierung auf das Auge zurückgebracht wird, welche sie vor dem Abtrennen eingenommen hat. Es erübrigt sich die nachträgliche Fixierung der zurückgeklappten Hornhaut, z.B. mit Hilfe von Nähten, welche bei einer vollständigen Abtrennung der Hornhautlamelle notwendig ist. Auch das Wiederanwachsen des Gewebes des abgetrennten Lamellenteils an das am Auge verbliebene Gewebe erfolgt übergangslos ohne Narbenbildung.

Für die oszillierende Messerbewegung und für den Vor- und Rückschub des Supports sind zwei getrennte Antriebe vorgesehen. Der Antrieb des Messers erfolgt über eine separate Welle, und der Antrieb für den Support erfolgt ebenfalls über eine separate Welle. Jede der beiden Wellen wird von einem zugeordneten Motor angetrieben. Hierdurch wird gewährleistet, daß der oszillierende Antrieb des Messers und der Antrieb für die Vor- und Rückschubbewegung des Supports und damit die Vor- und Rückschubbewegung des daran oszillierend gelagerten Messers getrennt voneinander gesteuert werden können. In bevorzugter Weise sind die beiden Motore über flexible Wellen mit dem Support und dem Messer verbunden. Hierdurch wird gewährleistet, daß das Schneidinstrument in den Strahlengang eines Laserablationsgerätes zur Hornhautbehandlung bewegt werden kann, bevor die Laserbehandlung am Patientenauge vorgenommen wird. Der Patient braucht seine Stellung für die Behandlung nicht zu wechseln. Die Schneidbehandlung kann daher am Patientenauge, welches sich unter der Bestrahlungsoptik des Lasergerätes befindet, durchgeführt werden und nach dem Schneidvorgang kann das Schneidgerät entfernt werden. Die beiden Motore können jedoch auch in Form von Mikromotoren direkt am Schneidgerät vorgesehen sein.

Aufgrund der getrennten Antriebe für die Supportbewegung (Vorschub und Rückschub) sowie für die oszillierende Messerbewegung kann der Antrieb für den Supportvorschub in der Weise gesteuert werden, daß immer dann, wenn die oszillierende Messerbewegung sich im jeweiligen Umkehrpunkt der Bewegungsrichtung (Geschwindigkeit = 0) befindet, angehalten wird. Hierdurch wird erreicht, daß beim jeweiligen Stillstand der Messerbewegung, bei welchem kein Schneiden stattfindet, auch der Messervorschub stillsteht.

Als trägheitsfrei arbeitende Antriebseinrichtung für den Supportvorschub eignet sich ferner ein piezoelektrischer Linearantrieb, welcher bevorzugt am Basisring abgestützt ist. Dieser piezoelektrische Antrieb kann in herkömmlicher Weise ausgebildet sein (z.B. DE 38 25 587) und aus Piezoaktuatoren zusammengesetzt sein. Es können beispielsweise drei Piezoaktuatoren vorgesehen sein. Ein geeigneter Piezoantrieb besteht aus drei Piezokristallen, deren Fortbewegung und daran gekoppelte Linearbewegung des Supportantriebs sich mit der Fortbewegung einer Spannerraupe erklären läßt. Dabei dienen der vordere und der hintere beispielsweise als Klemmaktuatoren ausgebildete Piezokristall als Bremse und der mittlere beispielsweise als Vorschubaktuator ausgebildete Piezokristall der Fortbewegung. Die in einem mit dem Support verbundenen Gehäuse befindlichen Piezokristalle, insbesondere Piezokeramiken, bewegen sich dabei auf einer mit dem Basisring direkt oder indirekt verbundenen Unterlage, insbesondere Metallband oder Schiene vorwärts. Der erste Piezokristall hält die Unterlage fest, während sich der zweite Piezokristall (mittlere Piezokristall) ausdehnt. Damit sich die Anordnung bewegen kann, ist die durch den dritten Piezokristall gebildete zweite Bremse offen. Nach der Ausdehnung klemmt sich der dritte Piezokristall an der Unterlage, insbesondere dem Metallband, fest, d.h. die zweite Bremse wird geschlossen, während sich die erste Bremse, d.h. der erste Piezokristall öffnet und sich der zweite Piezokristall (mittlere Piezokristall) zusammenzieht. Dann wiederholt sich der Vorgang. Die Antriebsart kann auch kinematisch umgekehrt sein, d.h. das Gehäuse ist mit dem Basisring direkt oder indirekt verbunden und die Unterlage, beispielsweise das Metallband, bewegt sich und ist mit dem Support verbunden.

Auch die oszillierenden Messerbewegungen können durch einen Kristallschwinger erzeugt sein. Es erübrigt sich dann die Ankopplung des oszillierenden Messers und des linear anzutreibenden Supports über flexible mit Motoren verbundenen Wellen. Die beiden Antriebe können dann direkt am Schneidgerät vorgesehen sein.

Anhand der Figuren wird die Erfindung noch näher erläutert. Es zeigt:

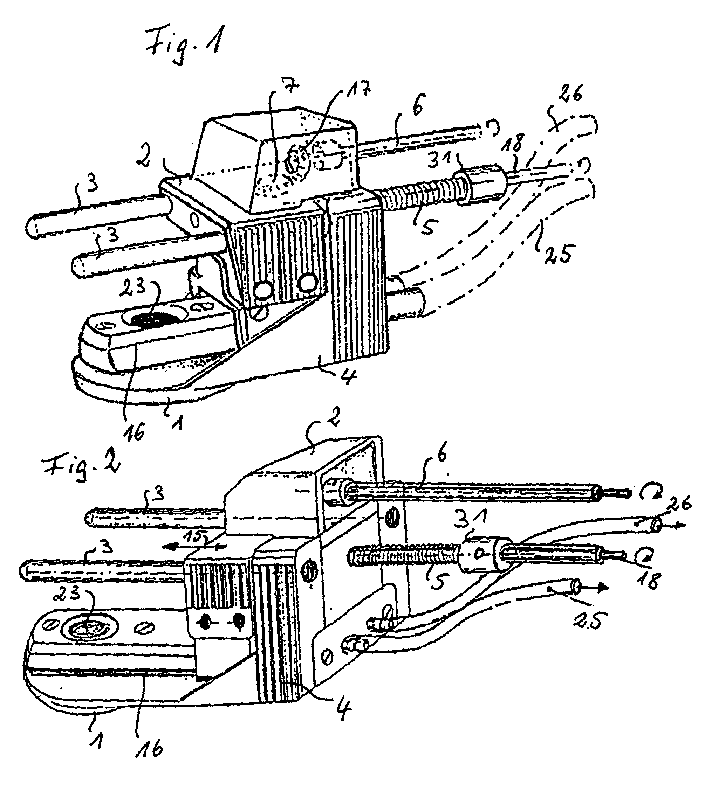

- Fig. 1:: ein Ausführungsbeispiel in perspektivischer Darstellung;
- Fig. 2:: das Ausführungsbeispiel in einer anderen perspektivischen Darstellung;
- Fig. 3:: ein Ausführungsbeispiel für einen Piezoantrieb;
- Fig. 4:: eine schnittbildliche Darstellung zur Erläuterung des Schneidvorgangs;
- Fig. 5:: ein Ausführungsbeispiel, bei dem ein Schneidgerät gemäß den Figuren 1 bis 3 mit einem Lasergerät zur Durchführung chirurgischer Eingriffe an der Augenhomhaut kombiniert ist; und
- Fig. 6:: eine schematische Darstellung der Hornhautbehandlung nach dem Schnitt.

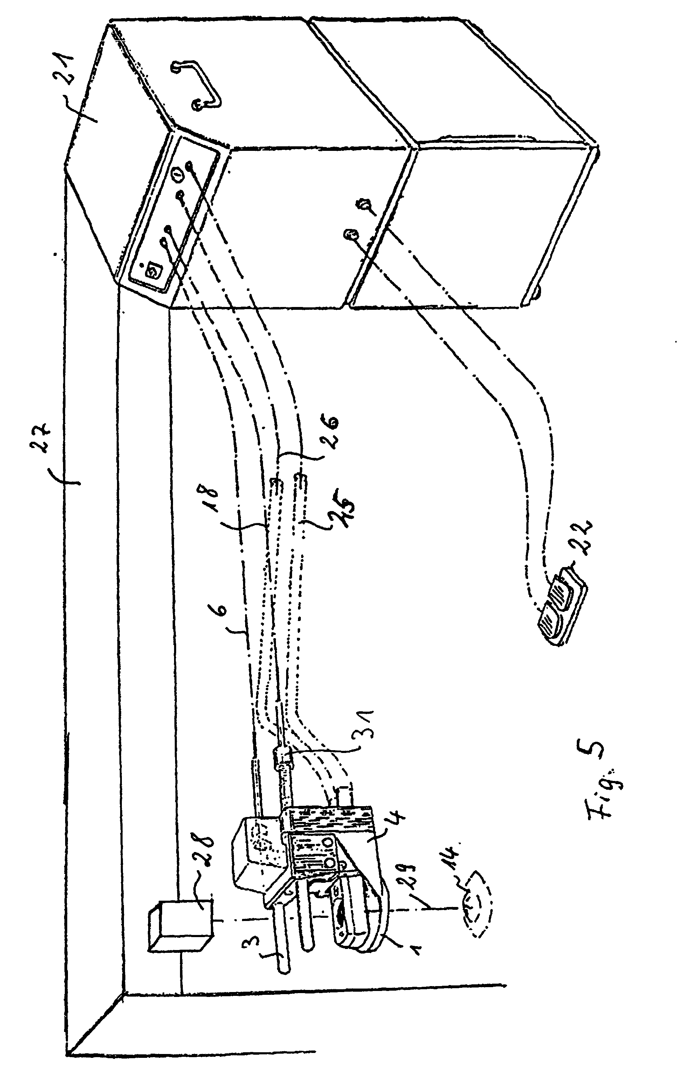

Das in den Figuren dargestellte Ausführungsbeispiel für ein Schneidgerät besitzt einen Basisring 1, der auf ein Patientenauge 14 (Fig. 5) aufsetzbar ist. Am Basisring 1 befindet sich an einem mit dem Basisring 1 fest verbundenen Gestell eine Supportführung in Form zweier zylindrischer Führungsstäbe 3. Die Führungsstäbe 3 erstrecken sich in der Vor- und Rückschubrichtung des Supportes 2. Im Support 2 ist das Messer in Form einer Klinge 11 oszillierend gelagert. Die Klinge 11 führt eine oszillierende Bewegung quer (vertikal) zur Vor- und Rückschubrichtung (Doppelpfeil 15) des Supportes aus. Beim Schneidvorgang wird die Klinge 11 in einer Klingenführung 16 an einem mit dem Basisring 1 verbundenen Gestell 4 geführt.

Für den Vor- und Rückschub des Supportes und der darin gelagerten Klinge 11 ist eine erste Antriebswelle 5 vorgesehen. Die Antriebswelle 5 besitzt ein Außengewinde, welches in ein entsprechendes Innengewinde des Supportes 2 für den Vor- und Rückschub eingreift.

Für den oszillierenden Antrieb der Klinge 11 ist eine zweite Antriebswelle 6 vorgesehen, welche an ihrem vorderen Ende ein Kegelrad 17 aufweist, das in ein zweites Kegelrad 7 eingreift und über ein nicht näher dargestelltes Getriebe in bekannter Weise, z.B. wie in der DE 34 33 581 dargestellt, mit Hilfe eines rotierenden Exzenters die oszillierende Bewegung der Klinge erzeugt.

Die erste Welle 5 ist über einen flexiblen Wellenstrang 18 mit einem zugeordneten ersten Antriebsmotor verbunden. Die zweite Antriebswelle 6 ist ebenfalls über einen flexiblen Wellenstrang mit einem zweiten Antriebsmotor verbunden. Die beiden Motoren können in einem zentralen Gehäuse 21 untergebracht sein. In diesem zentralen Gehäuse kann auch die Steuerungseinrichtung für weitere Komponenten, die bei der Hornhautbehandlung am Patientenauge 14 von Bedeutung sind, untergebracht sein. Beispielsweise kann ein Fußschalter 22 zum Ein- und Ausschalten bestimmter Komponenten, beispielsweise zur Steuerung des Supportvorschubs und des Supportrückschubs an die im Gehäuse 21 untergebrachte zentrale Steuerungseinrichtung angeschlossen sein.

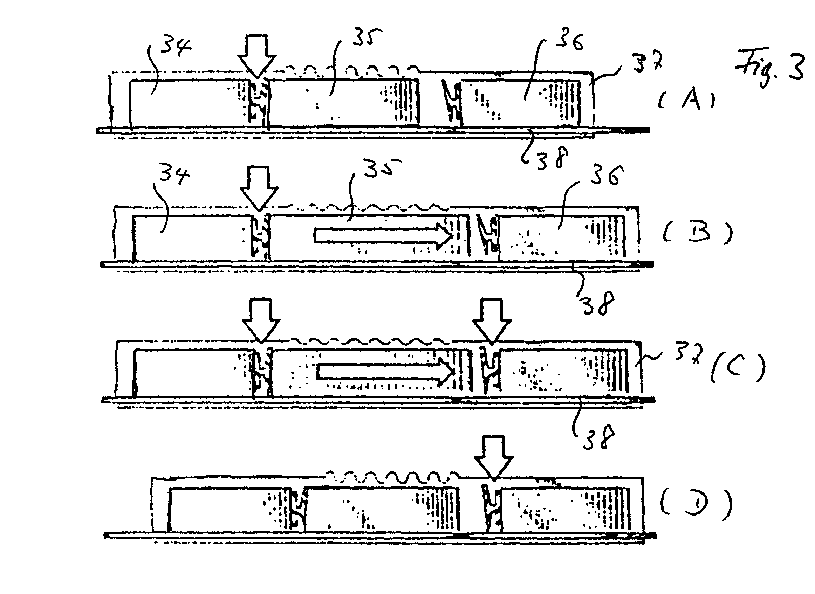

In der Fig. 3 ist ein Ausführungsbeispiel eines Piezoantriebs, insbesondere Linearantriebs, für den Support dargestellt. Dieser Linearantrieb besteht aus drei Piezokristallen 34, 35 und 36. Der vordere und der hintere Piezokristall 34 und 36 dienen als Bremse, und der mittlere Piezokristall 35 dient der Fortbewegung. Die drei Kristalle sind in einem Gehäuse 37 angeordnet, welches mit dem Support verbunden ist. Die Bewegung erfolgt auf einer Unterlage 38, welche als Band oder Schiene ausgebildet sein kann und direkt oder indirekt fest mit dem Basisring 1 verbunden ist. Beispielsweise kann eine der beiden Schienen 3 als Unterlage dienen. Hierdurch wird die Linearverschiebung des Supports 2 gegenüber dem Basisring 1 erzielt.

Bei der Bewegung des Gehäuses 37 und des damit verbundenen Supports 2 ist der erste Piezokristall 34 fest, beispielsweise durch Klemmen, mit der Unterlage 38 verbunden und wirkt als Feststellbremse. Der zweite Piezokristall 35 dehnt sich aus der in der Darstellung (A) gezeigten Position in die in der Darstellung B gezeigten Position aus, wobei der dritte Piezokristall 36 von der Unterlage 38 gelöst ist, so daß eine Verschiebung in die in den Darstellungen (B) und (C) gezeigten Positionen erfolgt. Nach der Ausdehnung des zweiten Piezokristalls 35 bis in die in der Darstellung (C) gezeigte Position wird der Piezokristall 37 fest mit der Unterlage 38 verbunden, während der erste Piezokristall 34 geöffnet wird und beim Zusammenziehen des zweiten Piezokristalls 35 nachrücken kann. Die drei Kristalle sind dann aus der in der Darstellung (A) gezeigten Anfangsposition bis in die in der Darstellung (D) gezeigte Position vorwärts bewegt worden, wobei auch gleichzeitig der mit dem Gehäuse 37 verbundene Support gegenüber der Unterlage 38 und damit gegenüber dem Basisring 1 mitbewegt worden ist. Anschließend wiederholt sich die Bewegungsfolge in einem nächsten Schritt.

Damit die Fortbewegung nach Art einer Spannerraupe möglich ist, ist das Gehäuse 37, insbesondere im Bereich des zweiten Piezokristalls 35, flexibel ausgebildet. Zur Steuerung der Bremsfunktionen des vorderen und hinteren Piezokristalls sowie zur Kontraktion und Expansion des zweiten Piezokristalls in der Fortbewegungsrichtung dienen bekannte Steuermittel, die beispielsweise in der DE 38 25 587 dargestellt sind.

Es ist natürlich auch möglich, das Gehäuse 37 mit dem Basisring zu verbinden und die Unterlagen 38 mit dem Support, so daß durch kinematische Umkehr die gleiche Linearbewegung für den Support erreicht werden kann. Beim Rückhub des Supportes werden die Bewegungsfolgen in umgekehrter Reihenfolge durch entsprechende Ansteuerung der Piezokristalle erreicht.

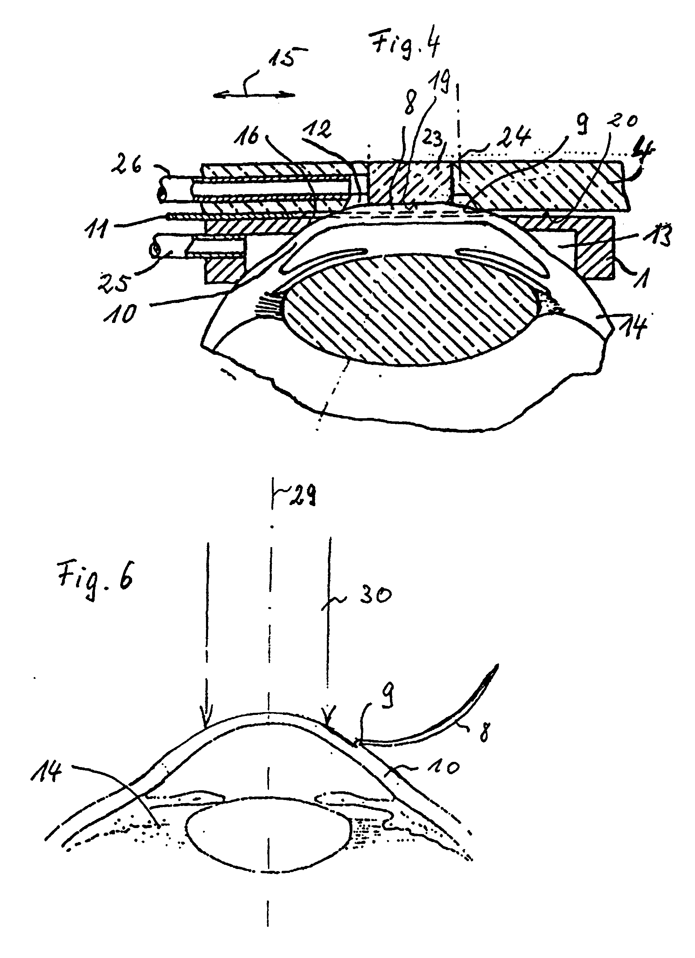

In der Figur 4 ist in schnittbildlicher Darstellung das auf das Patientenauge 14 aufgesetzte Schneidgerät dargestellt. Der Basisring 1 liegt auf der Augenoberfläche auf, und im Bereich des Augenbulbus wird zwischen dem Basisring 1 und der Augenoberfläche eine ringförmige Kammer 13 gebildet, die über eine druckdichte Leitung 25 an ein nicht näher dargestelltes Gerät zur Erzeugung eines Unterdrucks angeschlossen ist. Ferner wird im Bereich der Cornea eine Ringkammer 12 gebildet, die ebenfalls über eine druckdichte Leitung 26 an ein Gerät zur Erzeugung eines Unterdrucks angeschlossen ist. Die Geräte zur Unterdruckerzeugung können ebenfalls in dem Gehäuse 21 untergebracht sein. Zwischen dem Basisring 1 und dem Gestell 4 befindet sich die Klingenführung 16. Beim Vorschub bewegt sich die Klinge 11 in der Figur 4 von links nach rechts in der Führung 16. In dem Hohlraum der Führung 16 befindet sich die Schnittebene, in welcher eine Hornhautlamelle 8 vom Auge unter Beibehaltung eines Gewebescharniers 9 (Fig. 6) abgetrennt wird. Durch den in der Ringkammer 12 herrschenden Unterdruck wird die abzutrennende Hornhautlamelle während des Schnittvorgangs immer exakt fixiert. Oberhalb der durch Einschnitt teilweise abgetrennten Hornhautlamelle 8 befindet sich am Gestell 4 ein Schauglas 23. Im Schauglas 23 kann sich ein Fadenkreuz befinden, so daß eine exakte Ausrichtung des Schneidgerätes mit einer am Auge vor Durchführung des Schneidvorganges angebrachten Markierung exakt ausgerichtet werden kann.

An einem Gestellteil 4, das oberhalb der Klingenführung 16 angeordnet ist, ist an der Unterseite eine ebene Fläche 20 vorgesehen. Diese begrenzt die Klingenführung nach oben hin. Auch das Schauglas 23 hat an seiner Unterseite eine ebene Fläche 19. Diese ebene Fläche 19 ist gegenüber der ebenen Fläche 20 am Gestellteil 4 nach oben versetzt, so daß die gewünschte Schnittiefe, gemessen vom Hornhautscheitel senkrecht in das Hornhautgewebe, für die Hornhautlamelle eingestellt werden kann. Die ebene Fläche 19 an der Unterseite des Schauglases 23 verläuft parallel zu den beiden ebenen Flächen am Basisring 1 und am Gestellteil 4, welche die Klingenführung 16 begrenzen. Die Dicke der in abgeflachter Form unter der ebenen Fläche 19 des Schauglases 23 gehaltenen abzutrennenden Hornhautlamelle 8 läßt sich exakt einstellen. Bevorzugt wird eine Schnittiefe von 150 µm. Während des Schneidvorganges ist es von Vorteil, den Vorschub des Supports und damit des Messers immer dann anzuhalten, wenn die hin und her gehende Messerbewegung in ihren jeweiligen Umkehrpunkten (Geschwindigkeit =0) sich befindet.

In der Vorschubbahn der Klinge 11 ist eine Stelle vorgesehen, an welcher der Klingenvorschub bzw. Supportvorschub angehalten wird. Diese Stelle ist in der Fig. 4 mit 24 gekennzeichnet. Die Stelle 24, welche einen Bewegungsstop der Vorschubbewegung der Klinge bzw. des Supports kennzeichnet, ist so angeordnet und gegenüber dem zu behandelnden Auge 14 so festgelegt, daß durch den Schneidvorgang die von der Schnittebene in der Klingenführung 16 abgegrenzte Hornhautlamelle 8 nicht vollständig abgetrennt wird, sondern über das Gewebescharnier 9 mit der am Auge verbliebenen Hornhaut 10 verbunden bleibt.

Durch eine im Basisring 1 vorgesehene Öffnung, durch welche die Hornhaut ragt, wird eine Behandlungszone mit einem bestimmten Durchmesser, vorzugsweise von 9 mm, definiert. Diesen Durchmesser hat die Behandlungszone, welche in der Ebene der Klingenführung 16 liegt. Die über diese Ebene hinausragende Hornhautlamelle 8 wird während des Schnittvorgangs vom Auge teilweise abgetrennt, wobei, wie schon erläutert, der Schnittvorgang an der Bewegungsstopstelle 24 angehalten wird, so daß die abgetrennte Hornhautlamelle über das Gewebescharnier 9 mit dem Auge verbunden bleibt.

Zur Durchführung des Schneidvorgangs wird das Schneidgerät mit dem Basisring 1 auf das Auge aufgesetzt und durch Erzeugung eines Unterdruckes in der Kammer 13 fixiert. Ferner wird auch im Bereich der Cornea in der ringförmigen Kammer 12 ein Fixierungsunterdruck erzeugt. Auf diese Weise wird das Schneidgerät exakt auf dem Auge in der gewünschten Position fixiert. Das Einschalten der Unterdruckgeräte kann beispielsweise mit Hilfe des Fußschalters 22 erfolgen. Beispielsweise durch ein akustisches Signal kann der Bedienungsperson angezeigt werden, daß der gewünschte Unterdruck in den Ringkammern 12 und 13 vorhanden ist. Die hierzu verwendeten Pumpen können in den Kammern einen Druck von 0 bis 1 bar bilden.

Für den Schneidvorgang werden die beiden Antriebsmotore eingeschaltet, so daß die Klinge eine oszillierende Bewegung senkrecht zur Zeichenebene in Figur 4 und eine Vorschubbewegung von links nach rechts in der Figur 4 durchführt. Dabei wird die über die Ebene der Klingenführung 16 ragende Hornhautlamelle 8 abgetrennt, wobei jedoch der Schneidvorgang, wie schon erläutert, durch Anhalten des Klingenvorschubs an der Stelle 24 beendet wird.

Der Support 2 und die Klinge 11 werden dann zurückgefahren, wobei jedoch die bis zum Gewebescharnier 9 abgetrennte Hornhautlamelle 8 aufgrund des Unterdruckes in der Ringkammer 12 fixiert gehalten bleibt. Auch der Basisring 1 bleibt durch den in der Kammer 13 aufrechterhaltenen Unterdruck am Auge fixiert. Beim Zurückfahren der Klinge 11 wird der Antrieb für die oszillierende Schneidbewegung abgeschaltet. Dies kann durch Abschalten des der oszillierenden Schneidbewegung zugeordneten Motors geschehen oder durch Trennen einer nicht näher dargestellten Kupplung zwischen Motor und Antriebswelle 6. Die Rückschubbewegung des Supports und der Klinge 11 wird durch den Antrieb des anderen der Schubbewegung zugeordneten Motors vermittelt. Die Antriebsspindel 5 dreht sich dann In der zur Vorschubrichtung umgekehrten Drehrichtung.

Die oszillierende Klingenbewegung kann dann beendet werden bzw. abgeschaltet werden, wenn die Klinge die Bewegungsstopstelle 24 erreicht hat. Diese Bewegungsstopstelle kann durch entsprechende Steuerung und Anhalten des der Schubbewegung zugeordneten Motors in der zentralen Steuereinrichtung elektronisch festgelegt sein. Diese Stelle wird bestimmt, nachdem das Schneidgerät auf das Auge in der gewünschten Position aufgesetzt ist. Zusätzlich kann am Gerät ein zwischen dem Gestell 4 und dem Support 2 wirksamer Festanschlag zur Sicherung der Bewegungsstopstelle 24 vorgesehen sein. Ferner kann ein zwischen der Antriebsspindel 5 und dem flexiblen Wellenstrang 18 vorgesehenes Kupplungsstück 31 als Endanschlag dienen.

Nach Abschalten des Unterdruckes wird das Schneidgerät vom Auge abgenommen.

Beim Schneidvorgang beträgt die Oszillationsgeschwindigkeit der Klinge 1350 U/min. Die Vorschubgeschwindigkeit der Klinge beträgt 1,3 mm/s. Die Rückhubgeschwindigkeit bei abgeschalteter Oszillationsbewegung beträgt 3 mm/s. Durch die beiden getrennten Motore können die Oszillationsbewegung und die Vor- und Rückhubbewegung getrennt voneinander gesteuert werden. Durch die beiden zylindrischen Führungsstäbe 3 wird eine exakte Schnittführung der Klinge 11 am Basisring 1 und am Gestell 4 gewährleistet.

Wie insbesondere aus der Figur 5 zu ersehen ist, kann der Basisring 1 mit seiner Mitte unter Zuhilfenahme des im Schauglas 23 vorgesehenen Fadenkreuzes exakt auf die Hornhaut 10 aufgesetzt und dort mit Hilfe der Unterdruckkammern 12 und 13 fixiert werden. Hierdurch läßt sich gewährleisten, daß die Schneideinrichtung in den Bereich gebracht werden kann, in welchem der Strahlengang eines nach dem Schneidvorgang auf das Auge zu richtenden Laserstrahls liegt. Aufgrund der flexibel ausgebildeten Antriebswellen (flexibles Wellenstück 18 und flexible Welle 6), welche mit den ortsfest im Gehäuse 21 vorgesehenen Antriebsmotoren verbunden sind, läßt sich die Schneideinrichtung ungehindert an die gewünschte Position in der Laserbehandlungseinrichtung 27 bringen und von dort wieder entfernen. Das Gehäuse 21 mit den darin befindlichen Steuereinrichtungen kann in die die Laserbehandlungseinrichtung 27 zur Hornhautbehandlung, integriert sein. In diese Einrichtung wird in bekannter Weise der Patient mit seinem zu behandelnden Auge 14 unterhalb einer Bestrahlungsoptik 28 auf ein nicht näher dargestelltes Bett gelegt. Durch die Bestrahlungsoptik 28 wird entlang einer Fixierlinie 29 eine Laserstrahlung 30 auf das zu behandelnde Auge 14 gerichtet. Bei Kombination des Schneidgerätes mit der Einrichtung 27 erfolgt dies nach Durchführung des Schneidvorganges und nachdem die teilweise abgetrennte Hornhautlamelle 8 um die Scharnierstelle 9 zurückgeklappt ist und, wie in Figur 6 gezeigt ist, die Laserstrahlung 30 auf das freigelegte Stroma gerichtet werden kann. Mit Hilfe der Laserstrahlung können dann die gewünschten refraktiven Korrekturen am Auge 14 durchgeführt werden. Nach der Laserstrahlbehandlung wird die zurückgeklappte Hornhautlamelle 8 wieder in ihre vorherige Position auf die Hornhaut 10 zurückgebracht, welche exakt der vor der Behandlung vorhandenen Position am Auge entspricht.

## Patentansprüche

1. Vorrichtung zur Hornhautchirurgie mit einem auf das zu behandelnde Auge (14) aufsetzbaren und durch Unterdruck fixierbaren Basisring (1), einer Ringkammer (12), in der im Bereich der Hornhaut ein Unterdruck erzeugbar ist, einem oszillierenden Messer (11), das mittels eines auf dem Basisring (1) geführten Supports (2) über das Auge (14) bewegbar ist, und jeweils einem Antrieb für die oszillierende Messerbewegung und die Supportbewegung, wobei der Antrieb für die oszillierende Messerbewegung und der Antrieb für die Supportbewegung in unabhängig voneinander steuerbaren Antriebseinrichtungen (5, 6) bestehen, wobei die Vorrichtung eine zentrale Steuereinrichtung zum elektronischen Steuern und Anhalten des der Supportbewegung zugeordneten Antriebs (5) und des Antriebs (6) der Messerbewegung aufweist,
**dadurch gekennzeichnet,**
**daß** durch die zentrale Steuereinrichtung ein Bewegungsstop für den Supportvorschub während des Schneidvorgangs an einer Stelle (24) definiert wird, an welcher das von der Hornhaut abgetrennte lamelläre Hornhautstück (8) mit der am Auge (14) verbliebenen Hornhaut über eine vom Hornhautgewebe gebildete Scharnierstelle (9) verbunden ist, so daß das abgetrennte lamelläre Hornhautstück (8) zum Freilegen des darunter befindlichen Augengewebes seitlich wegklappbar ist und daß beim Rückhub des Supports (2) von der Stelle (24) des Bewegungsstops der Antrieb (6) der Messerbewegung durch die zentrale Steuereinrichtung anhaltbar ist.

2. Vorrichtung nach Anspruch 1, **dadurch gekennzeichnet, daß** beim Rückhub des Supports (2) der Basisring (1) durch Unterdruck am Auge (14) fixiert bleibt, wobei die Regelung des Unterdrucks durch die zentrale Steuereinrichtung erfolgt.

3. Vorrichtung nach Anspruch 1 oder 2, **dadurch gekennzeichnet, daß** beim Rückhub des Supports (2) der Unterdruck in der Ringkammer (12) im Bereich der Hornhaut aufrechterhalten bleibt, wobei die Regelung des Unterdrucks durch die zentrale Steuereinrichtung erfolgt.

4. Vorrichtung nach einem der Ansprüche 1 bis 3, **dadurch gekennzeichnet, daß** der Antrieb (5) für den Supportvorschub jeweils in den Umkehrpunkten der oszillierenden Messerbewegung durch die zentrale Steuereinrichtung anhaltbar ist.

5. Vorrichtung nach einem der Ansprüche 1 bis 4, **dadurch gekennzeichnet, daß** für den Antrieb (5) des Supports (2) und für den oszillierenden Antrieb (6) des Messers (11) jeweils ein zugeordneter Motor vorgesehen ist.

6. Vorrichtung nach einem der Ansprüche 1 bis 5, **dadurch gekennzeichnet, daß** der Basisring (1) nach Abschalten des Unterdrucks vom Auge (14) abhebbar ist.

7. Vorrichtung nach einem der Ansprüche 1 bis 6, **dadurch gekennzeichnet, daß** der Bewegungsstop (24) im Supportvorschub so angeordnet ist, daß an der Scharnierstelle (9) das Hornhautgewebe eine Dicke von etwa 0,3 mm aufweist.

8. Vorrichtung nach einem der Ansprüche 1 bis 7, **dadurch gekennzeichnet, daß** der Bewegungsstop (24) im Supportvorschub durch einen Festanschlag gesichert ist.

9. Vorrichtung nach einem der Ansprüche 1 bis 8, **dadurch gekennzeichnet, daß** die Schnitttiefe senkrecht in die Hornhaut so bemessen ist, daß das Stroma freigelegt ist.

10. Vorrichtung nach einem der Ansprüche 1 bis 9, **dadurch gekennzeichnet, daß** die über die Ebene einer Klingenführung (16), die parallel zur Ebene des Basisrings (1) liegt, hinausragende und abzutrennende Hornhautlamelle (8) an einer ebenen Fläche (19) anliegt, die parallel zur Ebene der Klingenführung (16) verläuft und entsprechend der Schnitttiefe gegenüber der Ebene der Klingenführung (16) versetzt ist.

11. Vorrichtung nach einem der Ansprüche 1 bis 10, **dadurch gekennzeichnet, daß** für den Vorschub des Supports (2) eine erste Antriebswelle (5) und für die oszillierende Messerbewegung eine zweite Antriebswelle (6) vorgesehen ist.

12. Vorrichtung nach Anspruch 11, **dadurch gekennzeichnet, daß** die erste Antriebswelle (5) von einem ersten Motor und die zweite Antriebswelle (6) von einem zweiten Motor angetrieben sind.

13. Vorrichtung nach Anspruch 11 oder 12, **dadurch gekennzeichnet, daß** die Antriebswellen (5, 6) aus einem flexiblen Material bestehen.

14. Vorrichtung nach Anspruch 12 oder 13, **dadurch gekennzeichnet, daß** die Motoren in einem zentralen Gehäuse (21) angeordnet sind.

15. Vorrichtung nach einem der Ansprüche 1 bis 12, **dadurch gekennzeichnet, daß** die beiden Antriebe für die Supportbewegung und die oszillierende Messerbewegung am Basisring (1) gelagert sind.

16. Vorrichtung nach Anspruch 15, **dadurch gekennzeichnet, daß** der Support (2) von einem piezoelektrischen Linearantrieb (34, 35, 36) angetrieben ist.

17. Vorrichtung nach Anspruch 16, **dadurch gekennzeichnet, daß** der piezoelektrische Linearantrieb (34, 35, 36) des Supports (2) am Basisring (1) abgestützt ist.

18. Vorrichtung nach Anspruch 15, **dadurch gekennzeichnet, daß** ein Schwingkristall, welcher die oszillierende Bewegung des Messers erzeugt, am Support (2) abgestützt ist.

19. Vorrichtung nach einem der Ansprüche 1 bis 14, **dadurch gekennzeichnet, daß** der Basisring (1) mit dem darauf geführten Support (2) mit einem Laserablationsgerät (27) derart kombiniert ist, daß der Basisring (1) mit seiner Mitte in den Bereich bewegbar ist, in welchem der bei der Laserablation auf das freigelegte Stroma gerichtete Laserstrahl (29) verläuft.

## Claims

1. Device for corneal surgery including a base ring (1) fittable onto the eye (14) to be treated and fixable by underpressure, a ring chamber (12), in which an underpressure is able to be generated in the region of the cornea, an oscillating knife (11) being movable across the eye (14) by means of a support (2) guided on the base ring (1), and one drive for the oscillating knife movement and the support movement, respectively, wherein the drive for the oscillating knife movement and the drive for the support movement exist in driving means (5, 6) controllable independently of each other, wherein the device has a central controller for electronically controlling and stopping the drive (5) associated with the support movement and the drive (6) of the knife movement,
**characterized in**
**that** by the central controller, a movement stop for the support feed during the cutting operation is defined at a location (24), at which the lamellar corneal piece (8) separated from the cornea is connected to the cornea left at the eye (14) through a hinge location (9) formed by the corneal tissue, such that the separated lamellar corneal piece (8) is able to be laterally folded away for exposing the underlying eye tissue, and that in the return stroke of the support (2) from the location (24) of the movement stop, the drive (6) of the knife movement is stoppable by the central controller.

2. Device according to claim 1, **characterized in that** in the return stroke of the support (2), the base ring (1) remains fixed to the eye (14) by underpressure, wherein the control of the underpressure is effected by the central controller.

3. Device according to claim 1 or 2, **characterized in that** in the return stroke of the support (2), the underpressure in the ring chamber (12) is maintained in the region of the cornea, wherein the control of the underpressure is effected by the central controller.

4. Device according to any one of claims 1 to 3, **characterized in that** the drive (5) for the support feed is stoppable respectively in the reversal points of the oscillating knife movement by the central controller.

5. Device according to any one of claims 1 to 4, **characterized in that** for the drive (5) of the support (2) and for the oscillating drive (6) of the knife (11), respectively, an associated motor is provided.

6. Device according to any one of claims 1 to 5, **characterized in that** the base ring (1) is able to be lifted from the eye (14) after shutting-off the underpressure.

7. Device according to any one of claims 1 to 6, **characterized in that** the movement stop (24) in the support feed is disposed such that the corneal tissue has a thickness of about 0,3 mm at the hinge location (9).

8. Device according to any one of claims 1 to 7, **characterized in that** the movement stop (24) in the support feed is secured by a fixed stop.

9. Device according to any one of claims 1 to 8, **characterized in that** the cutting depth perpendicularly into the cornea is dimensioned such that the stroma is exposed.

10. Device according to any one of claims 1 to 9, **characterized in that** the corneal lamella (8) protruding beyond the plane of a blade guide (16) lying parallel to the plane of the base ring (1), and to be separated abuts a flat surface (19) passing parallel to the plane of the blade guide (16) and being offset with respect to the plane of the blade guide (16) according to the cutting depth.

11. Device according to any one of claims 1 to 10, **characterized in that** there are provided a first drive shaft (5) for the feed of the support (2), and a second drive shaft (6) for the oscillating knife movement.

12. Device according to claim 11, **characterized in that** the first drive shaft (5) is driven by a first motor, and the second drive shaft (6) is driven by a second motor.

13. Device according to claim 11 or 12, **characterized in that** the drive shafts (5, 6) are made of a flexible material.

14. Device according to claim 12 or 13, **characterized in that** the motors are disposed in a central housing (21).

15. Device according to any one of claims 1 to 12, **characterized in that** the two drives for the support movement and the oscillating knife movement are supported on the base ring (1).

16. Device according to claim 15, **characterized in that** the support (2) is driven by a piezoelectric linear drive (34, 35, 36).

17. Device according to claim 16, **characterized in that** the piezoelectric linear drive (34, 35, 36) of the support (2) is supported on the base ring (1).

18. Device according to claim 15, **characterized in that** an oscillating crystal producing the oscillating movement of the knife is supported on the support (2).

19. Device according to any one of claims 1 to 14, **characterized in that** the base ring (1) with the support (2) guided thereon is combined with a laser ablation apparatus (27) such that the base ring (1) is movable with its center into the region, in which the laser beam (29) directed to the exposed stoma in laser ablation passes.

## Revendications

1. Dispositif de chirurgie cornéenne comprenant un anneau de base (1) pouvant être mis en place sur l'oeil (14) à traiter et fixable par dépression, une chambre annulaire (12) dans laquelle une dépression peut être créée au droit de la cornée, un couteau oscillant (11) adapté pour passer sur l'oeil (14) moyennant un support (2) guidé sur l'anneau de base (1), ainsi qu'un mécanisme d'entraînement chacun pour le mouvement oscillant du couteau et le mouvement du support, lesdits mécanismes d'entraînement relatifs aux mouvement oscillant du couteau et du support étant constitués par des dispositifs de commande (5, 6) réglables indépendamment l'un de l'autre, le dispositif comprenant une unité de commande centrale destinée à commander et arrêter le mécanisme d'entraînement (5) associé au mouvement du support ainsi que le mécanisme d'entraînement (6) associé au mouvement du couteau,
**caractérisé en ce que** grâce à l'unité de commande centrale, un arrêt de l'avance du support pendant l'opération de coupe est défini à un endroit (24) auquel le lambeau de cornée (8) lamellaire enlevé de la cornée se trouve relié à la partie de la cornée restée sur l'oeuil (14) par l'intermédiaire d'un point de charnière (9) formé par le tissu de cornée, de manière que le lambeau de cornée (8) lamellaire enlevé peut être replié vers le côté et que lors de la course de retour du support (2) depuis l'endroit (24) d'arrêt de l'avance, le mécanisme d'entraînement (6) du mouvement du couteau peut être arrêté grâce à l'unité de commande centrale.

2. Dispositif selon la revendication 1, **caractérisé en ce que** lors de la course de retour du support (2), l'anneau de base (2) reste fixé sur l'oeil (14) par dépression, le réglage de la dépression étant assuré par l'unité de commande centrale.

3. Dispositif selon la revendication 1 ou 2, **caractérisé en ce que** lors de la course de retour du support (2), la dépression dans la chambre annulaire (12) au droit de la cornée est maintenue, le réglage de la dépression étant assuré par l'unité de commande centrale.

4. Dispositif selon l'une des revendications 1 à 3, **caractérisé en ce que** le mécanisme d'entraînement (5) relatif à l'avance du support est susceptible d'être suspendu aux points de retour du mouvement oscillant du couteau respectivement, à l'aide de l'unité de commande centrale.

5. Dispositif selon l'une des revendications 1 à 4, **caractérisé en ce que** pour l'entraînement (5) du support (2) et l'entraînement oscillant (6) du couteau (11), un moteur associé est prévu chacun.

6. Dispositif selon l'une des revendications 1 à 5, **caractérisé en ce que** l'anneau de base (1) est apte à être enlevé de l'oeil (14) après suspension de la dépression.

7. Dispositif selon l'une des revendications 1 à 6, **caractérisé en ce que** l'arrêt (24) de l'avance du support est disposé de façon que le tissu cornéen présente, au droit du point de charnière (9), une épaisseur d'environ 0,3 mm.

8. Dispositif selon l'une des revendications 1 à 7, **caractérisé en ce que** l'arrêt de l'avance (24) du support est obtenu à l'aide d'une butée fixe.

9. Dispositif selon l'une des revendications 1 à 8, **caractérisé en ce que** la profondeur de la coupe verticale dans la cornée est dimensionnée de façon à ce que le stroma soit mis à nu.

10. Dispositif selon l'une des revendications 1 à 9, **caractérisé en ce** la lamelle cornéenne (8) à enlever et faisant saillie au-dessus du plan d'un guidage de la lame (16) s'étendant parallèlement au plan de l'anneau de base (1) s'applique contre une surface plane (19) s'étendant parallèlement au plan du guidage de la lame (16) et, en fonction de la profondeur de coupe, décalée par rapport au plan de guidage de la lame (16).

11. Dispositif selon l'une des revendications précédentes 1 à 10, **caractérisé en ce que** pour l'avance du support (2), un premier arbre de commande (5) est prévu et pour le mouvement oscillant du couteau, un second arbre de commande (6) est prévu.

12. Dispositif selon la revendication 11, **caractérisé en ce que** le premier arbre de commande (5) est entraîné par un premier moteur, le second arbre de commande (6) étant entraîné par un second moteur.

13. Dispositif selon la revendication 11 ou 12, **caractérisé en ce que** les arbres de commande (5, 6) sont réalisés en un matériau flexible.

14. Dispositif selon la revendication 12 ou 13, **caractérisé en ce que** les moteurs sont disposés dans un carter central (21).

15. Dispositif selon l'une des revendications 1 à 12, **caractérisé en ce que** les deux mécanismes d'entraînement relatifs au mouvement du support et au mouvement oscillant du couteau sont logés au niveau de l'anneau de base (1).

16. Dispositif selon la revendication 15, **caractérisé en ce que** le support (2) est entraîné à l'aide d'un mécanisme d'entraînement linéaire (34, 35, 36) piézoélectrique.

17. Dispositif selon la revendication 16, **caractérisé en ce que** le mécanisme d'entraînement linéaire (34, 35, 36) piézoélectrique du support s'appuie sur l'anneau de base (1).

18. Dispositif selon la revendication 15, **caractérisé en ce qu'**un cristal oscillant générant le mouvement oscillant du couteau s'appuie sur le support (2).

19. Dispositif selon l'une des revendications 1 à 14, **caractérisé en ce que** l'anneau de base (1) comprenant le support (2) guidé là-dessus est combiné à un appareil d'ablation à laser (27) de la sorte que le centre de l'anneau de base (1) puisse être déplacé jusqu'à l'endroit où s'étend le rayon laser (29) dirigé sur le stroma mis à nu pendant l'ablation à laser.
